# EUROPÄISCHE PATENTSCHRIFT

(11) **EP 0 743 932 B1**
(45) Veröffentlichungstag und Bekanntmachung des Hinweises auf die Patenterteilung: **28.10.1998**
(21) Anmeldenummer: 95907645.6
(22) Anmeldetag: 06.02.1995
(51) Int. Cl.: C07C 17/25, C07C 21/06

(54) **VERFAHREN ZUR HERSTELLUNG VON VINYLCHLORID**
PROCESS FOR PRODUCING VINYL CHLORIDE
PROCEDE DE PRODUCTION DE CHLORURE DE VINYLE

(30) Priorität: 12.02.1994 DE 4404510
(43) Veröffentlichungstag der Anmeldung: 27.11.1996
(73) Patentinhaber: HOECHST AKTIENGESELLSCHAFT, 65926 Frankfurt am Main (DE)
(72) Erfinder: SCHWARZMAIER, Peter, D-84556 Kastl (DE); HÄCKL, Peter, D-84556 Kastl (DE); STÖGER, Manfred, D-84508 Burgkirchen (DE); MIELKE, Ingolf, D-84508 Burgkirchen (DE)
(74) Vertreter: Schuderer, Michael
(86) Internationale Anmeldenummer: EP9500410
(87) Internationale Veröffentlichungsnummer: WO9521807

(56) Entgegenhaltungen:
- EP-A- 0 027 554
- DE-A- 4 142 117

## Beschreibung

Vinylchlorid (VC) wird durch thermische Abspaltung von Chlorwasserstoff aus 1,2-Dichlorethan (EDC) erhalten, wobei der Umsatz üblicherweise in der Größenordnung von 60 % gehalten wird, um die Bildung von Nebenprodukten und insbesondere von Koks gering zu halten. Ein Umsatz von etwa 60 % bedeutet, daß das aus der Spaltung hervorgehende Gasgemisch - im folgenden "Spaltgas" genannt - im wesentlichen ein ternäres Gemisch aus VC, Chlorwasserstoff und EDC ist.

Eine Bestimmung des Spaltungsumsatzes durch Ermittlung der VC-Konzentration im Spaltgas ist mit erheblichen Schwierigkeiten verbunden, da Anschlüsse für analytische Bestimmungen wie extraktive Messungen oder beispielsweise für Gaschromatographen in kurzer Zeit durch Verlegungen zugesetzt werden. In der Praxis begnügt man sich deshalb meistens mit einer Messung der Temperatur am Ausgang des Spaltofens, wobei dieser Temperaturmeßwert zur Regelung der Heizung, beispielsweise der Methanzufuhr, herangezogen wird. Auf diese Weise wird die Ausgangstemperatur möglichst konstant gehalten.

Es wurde gefunden, daß durch äußere Einflüsse auch bei konstanter Ausgangstemperatur am Spaltofen eine gewisse Streubreite beim EDC-Umsatz von etwa ± 2 % auftritt. Dies verhindert die Einhaltung optimaler Reaktionsbedingungen und die Minimierung der Nebenproduktbildung und des Energieverbrauchs.

Der Erfindung liegt die Aufgabe zugrunde, den Umsatz im EDC-Spaltofen möglichst konstant zu halten und dadurch die Bildung von Nebenprodukten und insbesondere die Koksbildung zu unterdrücken. Diese Aufgabe wird erfindungsgemäß dadurch gelöst, daß als Regelgröße die Absorption energiereicher, insbesondere radioaktiver Strahlung, zweckmäßig am Ausgang des Spaltofens, bestimmt wird. Es wurde nämlich gefunden, daß die gemessene Absorption ein Maß für die Dichte des Spaltgases und die VC-Konzentration ist. Sofern gewünscht, kann der Meßwert nach entsprechender Kalibrierung also auch als Dichte des Spaltgases beziehungsweise als VC-Konzentration ausgedrückt werden. Diese Meßgröße kann also unmittelbar zur Regelung der Brennstoffzufuhr für die Beheizung des Spaltofens herangezogen werden.

Die erfindungsgemäße Umsatzbestimmung ist vor allem dann vorteilhaft, wenn für mehrere, parallel betriebene Spaltöfen eine gemeinsame Aufarbeitung stattfindet. Man kann so bei einer Schwankung im Umsatz ohne weiteres feststellen, in welchem von mehreren Öfen die Umsetzung nicht programmgemäß verläuft.

Die Erfindung erlaubt die exakte Ermittlung des Umsatzes und seine Einhaltung beziehungsweise Regelung. Sie bewirkt damit eine zuverlässigere Unterdrückung der Nebenproduktbildung, insbesondere der Koksbildung, was wiederum zu erheblich verlängerten Standzeiten der gesamten Anlage führt.

Die radiometrische Messung der Schaumdichte wurde bereits zur Bestimmung des Umsatzgrades einer Polymerisation herangezogen (DE-A 41 42 117). Hierzu wird eine Latex-Probe aus dem Polymerisationskessel in einem Meßrohr entspannt und der dabei gebildete Schaum radiometrisch durchstrahlt. Die beim Durchtritt durch das zu messende Medium eintretende Schwächung von γ-Strahlen ist gemäß dem Lambert-Beerschen Gesetz ein Maß für die Dichte. Bei konstantem Meßweg ist die noch am Detektor ankommende Reststrahlung somit ein Maß für die Dichte des zu messenden Produktes. Allerdings erlaubt dieses beschriebene Meßverfahren keine kontinuierliche Überwachung des Umsatzes.

Die erfindungsgemäße radiometrische Messung am Spaltgas wird wie folgt vorgenommen: Als Dichtemeßstrecke dient ein wärmeisoliertes S-förmiges Rohr einer Länge von circa 3000 mm. Es wurde nämlich gefunden, daß in den Rohrbögen dort nur so geringe Ablagerungen auftreten, daß die Messung nicht beeinträchtigt ist. Radioaktiver Strahler, insbesondere ein γ-Strahler, und
Empfänger werden deshalb im Bereich der Rohrbögen - gegenüberliegend - installiert. Gleichzeitig werden in diesem Rohrabschnitt Druck und Temperatur gemessen. Die Meßwerte "Betriebsdichte", Druck und Temperatur werden in einem Rechner kontinuierlich erfaßt und über Berechnungsalgorithmen zur Normdichte des Spaltgases korrigiert. Aus dieser Normdichte kann im Rechner der Umsatz bestimmt werden.

Die erfindungsgemäße Bestimmung des EDC-Umsatzes ist unabhängig von der Durchführung der EDC-Spaltung. Diese erfolgt zweckmäßig unter Rückgewinnung der Spaltgaswärme, beispielsweise nach den Verfahren der EP-A 21 381, 264 065 oder 276 775.

Die Erfindung wird im folgenden Beispiel näher erläutert.

### Beispiel

Das aus dem EDC-Spaltofen austretende Spaltgas (bestehend im wesentlichen aus EDC, VC, HCl, jedoch auch Spuren von Nebenprodukten) wird in ein wärmeisoliertes, S-förmig gebogenes, circa 3000 mm langes Rohrstück geleitet. An gegenüberliegenden Rohrkrümmungen sind Sender und Empfänger für γ-Strahlung montiert.

Die laufend gemessene Absorption der γ-Strahlung, der Druck und die Temperatur werden einer Datenverarbeitungsanlage zugeführt, wo aus diesen Meßwerten die Betriebsdichte ermittelt wird. In einem Analog-Digitalwandler wird die Betriebsdichte zur Normdichte korrigiert. Über ein weiteres iteratives Rechenverfahren wird daraus der Umsatz bestimmt. Dieser dient als Regelgröße für die Brennstoffzufuhr.

## Patentansprüche

1. Verfahren zur Herstellung von Vinylchlorid durch thermische Spaltung von 1,2-Dichlorethan, dadurch gekennzeichnet, daß im Gasstrom, der aus dem Spaltofen austritt, durch Messung der Absorption energiereicher Strahlung, der Temperatur und des Druckes eine Meßgröße bestimmt wird, die zur Regelung der Umsetzung dient.

2. Verfahren nach Anspruch 1, dadurch gekennzeichnet, daß als energiereiche Strahlung radioaktive Strahlung dient.

3. Verfahren nach Anspruch 1 oder 2, dadurch gekennzeichnet, daß die beim Durchtritt durch das Spaltgas eintretende Schwächung von γ-Strahlen gemessen wird.

4. Verfahren nach einem oder mehreren der Ansprüche 1 bis 3, dadurch gekennzeichnet, daß die Regelung der Umsetzung über die Brennstoffzufuhr für den Spaltofen erfolgt.

## Claims

1. Process for producing vinyl chloride by thermal cracking of 1,2-dichloroethane, characterized in that a parameter in the gas stream issuing from the cracking furnace is determined by measuring the absorption of high-energy radiation, the temperature and the pressure, which parameter serves for control of the reaction.

2. Process according to Claim 1, characterized in that radioactive radiation serves as the high-energy radiation.

3. Process according to Claim 1 or 2, characterized in that the attenuation of γ-rays occurring on transmission through the cracking gas is measured.

4. Process according to one or more of Claims 1 to 3, characterized in that the reaction is controlled via the fuel supply to the cracking furnace.

## Revendications

1. Procédé de fabrication de chlorure de vinyle par craquage thermique de 1,2-dichloréthane, caractérisé en ce qu'on détermine dans le courant de gaz qui sort du four de craquage, par mesure de l'absorption d'un rayonnement riche en énergie, de la température et de la pression, une grandeur de mesure qui sert au réglage du craquage.

2. Procédé selon la revendication 1, caractérisé en ce qu'un rayonnement radioactif sert de rayonnement riche en énergie.

3. Procédé selon la revendication 1 ou 2, caractérisé en ce que l'on mesure l'atténuation de rayons γ se produisant lors du passage à travers du gaz de craquage.

4. Procédé selon une ou plusieurs des revendications 1 à 3, caractérisé en ce que le réglage du craquage s'effectue par l'acheminement de carburant au four de craquage.
